# EUROPEAN PATENT APPLICATION

(11) **EP 1 774 929 A2**
(43) Date of publication of application: **18.04.2007**
(21) Application number: 06122135.4
(22) Date of filing: 11.10.2006
(51) Int. Cl.: A61F 5/00

(54) **Overweight control apparatuses for insertion into the stomach**

(30) Priority: 12.10.2005 US 250008
(71) Applicant: Hull, Sr., Wendell C., Las Cruces, New Mexico 88011 (US); Hull, Jr., Wendell C., Cincinnati, Ohio 45241 (US)
(72) Inventor: Hull, Sr., Wendell C., Las Cruces, New Mexico 88011 (US); Hull, Jr., Wendell C., Cincinnati, Ohio 45241 (US)
(74) Representative: Kupecz, Arpad

(57) **Abstract**

A gastric balloon apparatus. An apparatus is disclosed that is insertable into a patent's stomach for treatment of overweight. The balloon occupies a volume of the gastric lumen to provide a sensation of fullness after the consumption of only modest amounts of food. The balloon apparatus has a basic toroidal shape to prevent blockage of the entrance or exit lumens of the stomach and promote proper passage of food through the stomach, while protecting the stomach lining from ulceration and irritation. A series of toroidal balloons of graduated diameter may be joined by inner and outer sleeves to define a funnel-shaped apparatus which expands when food is ingested, thus satiating the patient with substantially reduced quantity of food. A balloon storage and insertion apparatus also is disclosed, whereby a gastric balloon according to the disclosure may be pre-inflated and stored ion a tube for later use, whereupon the pre-inflated balloon is deployed into the stomach. Various mechanisms are disclosed for providing a pre-determined deflation of an inserted balloon, permitting the deflated balloon to be excreted from the body.

## Description

### BACKGROUND OF THE INVENTION

### Field of the Invention (Technical Field):

The present invention relates to methods and apparatus for treating overweight in humans, specifically to apparatuses insertable into a patient's stomach to reduce the patient's proclivity to overeat.

### Background Art:

During the past two decades, a means that has been pursued for overweight treatment in humans has been devices which were inserted into the stomach. These devices have taken various forms, including inflatable balloon systems. Typical prior art, as in the current disclosure, relate to balloons formed from thin elastic membranes that are inflated with a fluid, such as air or saline solution. The purpose of these balloons was to limit meal size, but allow ingestion of the amount of food required to maintain health with the goal of limiting weight gain or reducing weight.

These known devices are intended to effectively limit the available volume of the stomach for food. The aim is to achieve early satiety by imparting to the patient those sensations which normally would be experienced from eating a larger meal. However, prior art is limited in its ability to achieve appropriate and controlled limited fill, is not designed to reduce motility, and may have a tendency to cause obstruction. It is also limited in its use because of complex procedures required to implant in a patient, and maintain for the duration of the associated therapy. Prior art balloons typically are inserted into the stomach either transorally or percutaneously before they are inflated. The associated complexities of the insertion method, anatomically inadequate geometry, and the potentiality of the balloon to cause food obstruction in prior configurations have led to unsatisfactory performance and added health risks to the patient.

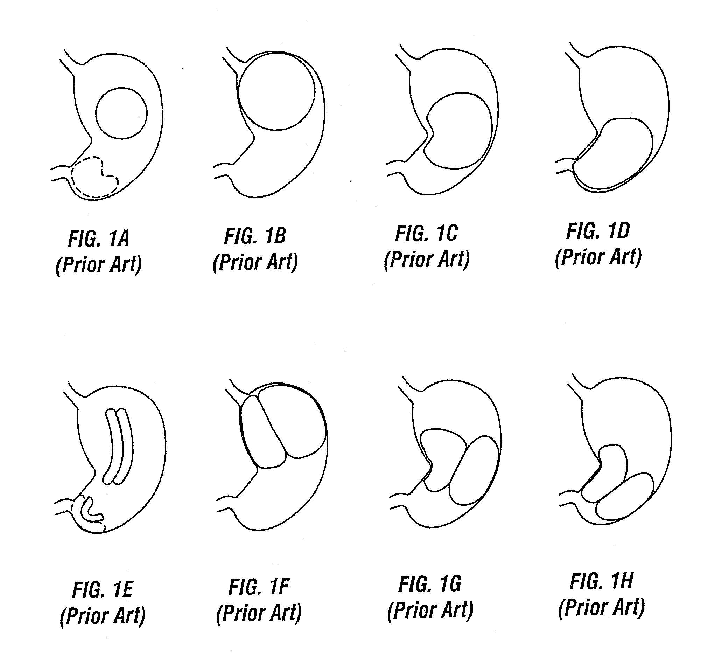

Figs. 1A-1H illustrate some of the difficulties associated with the prior art devices. Figs. 1A-1D show malfunctions of prior art spherical or (slight oval shape) balloons. In Fig. 1 A, a spherical balloon of low inflation is shown near the center of the stomach. It is noted that the relative amount of inflation of this balloon would not represent a very significant reduction in the available stomach volume, and hence foreseeably would not work well in providing a sense of early satiety to the patient.

It also is noted that for a saline-filled balloon, the balloon would likely settle into the lower part of the stomach by gravity and potentially be in a position, as shown by phantom lines in Fig. 1A, to interfere with food passage out the exit lumen of the stomach.

In the case of a spherical balloon that is inflated to a greater volume, as would be needed to provide the feeling of early satiety, the Figs. 1B-1D indicate alternate potential positions for the balloon after insertion. In Fig. 1B it is observed that the balloon is in a position which will allow it to provide local circumferential contact around the equator of the balloon, and to close off, or obstruct, the entrance lumen of the stomach. Hence food would have difficulty entering the stomach and, after entering, in passing beyond the balloon's equator. Additionally it's seen that a large portion of the inside surface of the stomach would not have sensual contact for purposes of satiety indication.

Fig. 1C shows the balloon spanning the mid portion of the stomach in the region of the gastric notch. In this case, localized circumferential contact at the balloon's equator with the inside surface of the stomach would again provide a food passage obstruction, and depending on the level of the balloon's internal pressure, a potential for enhancement of gastric lining ulceration. It is noted that again a very small proportion of the inner surface of the stomach would feel satiety contact.

Fig. 1D shows the balloon in the lower region of the stomach, a position the achievement of which would be enhanced by gravity. It can be seen again that the circumferential balloon equator contact with the inside of the stomach would provide a food passage obstruction. An interference, or stoppage, of the stomach's exit lumen is also indicated, as well as a low proportion of satiety contact as seen in Figs. 1B and 1C.

Malfunction modes of the other prior art balloon is depicted in Figs. 1E-1H. These figures show an elongated cylindrical toroidal balloon. Fig. 1E shows a low inflation balloon of this type. The similarity with Fig. 1A can be seen. Again the inflation volume would not be sufficient to contribute to early satiety, and the balloon through gravity could drop to the position shown in phantom lines and obstruct the stomach's exit lumen.

Figs. 1F-1H are analogous to Figs. 1B-D, with the associate discussion being the same except for the effect greater inflation of the balloon would have on the central opening in the balloon. As shown, the effect could be the closing off of the central passage through the balloon, which in turn would change the balloon into a "spherical" balloon for all practical purposes.

Thus, neither of the two prior art balloon devices functions effectively to provide both volume take-up and controlled stomach lining contact for early satiety in an adequate manner, but would lead to foreseeable risks for the patent.

Another objective of this disclosure is to provide a means of inserting and retrieving a gastric balloon apparatus without causing undue inconvenience or risk to the patient and to minimize costs associated with the use of the associated procedure. The apparatuses disclosed herein address all of the aforementioned goals and objectives. This is not true of the prior art devices.

### BRIEF DESCRIPTION OF THE DRAWINGS

The accompanying drawings, which are incorporated into and form a part of the specification, illustrate several embodiments of the present invention and, together with the description, serve to explain the principles of the invention. The drawings are only for the purpose of illustrating a preferred embodiment of the invention and are not to be construed as limiting the invention. In the drawings:
Figs. 1A-1H are side section views of prior art gastric balloon devices within the stomach of a patient;
Fig. 2A is a perspective view, from above, of a basic embodiment of the gastric balloon apparatus of this disclosure;
Fig. 2B is a side sectional view of the apparatus of Fig. 2A;
Fig. 3 is an enlarged sectional view of the time-release deflation mechanism of the apparatus shown in Fig. 2A;
Fig. 4 is a sectional side view of a plurality of gastric balloon apparatuses, similar to that shown in Figs. 2A-B, disposed within a patient's stomach;
Fig. 5A is a sectional side view of a gastric balloon apparatus similar that shown in Figs. 2A-B, with an alternative version of the time-release deflation mechanism;
Fig. 5B is an enlarged end or axial view, taken from plane B-B in Fig. 5A, of a portion of the gastric balloon apparatus, showing the alternative version of the time-release deflation mechanism seen in Fig. 5A;
Fig. 5C is an enlarged top view of the time-release deflation mechanism of the apparatus of Fig. 5A;
Fig. 5D is an enlarged side sectional view of a portion of the gastric balloon apparatus of Fig. 5A, showing the alternative version of the time-release deflation mechanism;
Fig. 6 is an enlarged sectional side view of an alternative, tube-type time-release deflation mechanism useable with the apparatus of this disclosure, similar to that shown in Fig. 3;
Fig. 7A is a sectional side view of a gastric balloon apparatus according to this disclosure, similar to that depicted in Figs. 2A-B, showing an alternative, annular gasket-type time-release deflation mechanism;
Fig. 7B is an enlarged end or axial view, taken from plane B-B in Fig. 7A, of a portion of the gastric balloon apparatus, showing the gasket-type time-release deflation mechanism of Fig. 7A;
Fig. 7C is an enlarged side sectional view of a portion of the gastric balloon apparatus of Fig. 7A, showing the alternative version of the time-release deflation mechanism;
Fig. 8A is a sectional side view of a gastric balloon apparatus according to this disclosure, similar to that depicted in Figs. 2A-B, showing another alternative, dimple-type time-release deflation mechanism;
Figs. 8B and 8C are enlarged sectional side views of portions of the apparatus seen in Fig. 8A, showing the dimple time-release mechanisms;
Fig. 9A is a perspective view, from above, of an embodiment of the gastric balloon apparatus of this disclosure, similar to that depicted in Figs 2A-B, showing another alternative, groove-type time release deflation mechanism;
Figs. 9B and 9C are enlarged sectional side views of portions of the apparatus seen in Fig. 9A, taken along section lines B-B, and C-C respectively, showing the groove time-release mechanisms in profile;
Fig. 10A is a sectional side view of a gastric balloon apparatus according to this disclosure, similar to that depicted in Figs. 2A-B, showing another alternative, string-type time-release deflation mechanism;
Fig. 10B is an enlarged side sectional view of a portion of the gastric balloon apparatus of Fig. 10A, showing the alternative version of the time-release deflation mechanism;
Figs. 10C and 10D are enlarged top view of the time-release deflation mechanism of the apparatus of Fig. 10A, showing possible alternative versions of the string-type time-release deflation mechanism of Fig. 10A;
Fig. 11A is a perspective view, from above, of an alternative embodiment of the gastric balloon apparatus of this disclosure, similar to that depicted in Figs 2A-B, showing optional structural spoke elements;
Fig. 11B is a sectional side view, taken on section line B-B of Fig. 11A, of the embodiment of Fig. 11A;
Fig. 12 is a sectional side view of an alternative embodiment of the apparatus of this disclosure, showing a funnel-like gastric balloon situated in the stomach of a patient;
Fig. 13 is a sectional side view of yet another alternative embodiment of the apparatus of this disclosure, showing two segments of funnel-like gastric balloon situated in the stomach of a patient;
Fig. 14 is a sectional side view of still another alternative embodiment of the apparatus of this disclosure, showing three segments of funnel-like gastric balloon situated in the stomach of a patient;
Fig. 15 is a sectional side view of a gastric balloon apparatus similar to that depicted in Fig. 12, showing a manner and mode of fabricating the apparatus;
Fig. 16A is a sectional side view of still another embodiment of a gastric balloon apparatus according to the present disclosure;
Fig. 16B is an exploded view, in reduced scale, of the apparatus seen in Fig. 16A, illustrating the fabrication of the apparatus from a stacked series of membrane sheets;
Fig. 16 C is a side sectional view, at reduced scale, of the embodiment of the apparatus seen in Fig. 16A, shown in an inflated condition;
Fig. 16D is a perspective view, from above and reduced in scale, of the embodiment apparatus shown in Fig. 16C;
Fig. 17A is a side sectional view of a gastric balloon storage and insertion apparatus, showing a gastric balloon collapsed within the insertion tube;
Fig. 17B is a subsequent view of the apparatus of Fig. 17A, illustrating the gastric balloon inflated by the insertion of filler fluid to expand within the balloon storage and insertion apparatus;
Fig. 17C is a view of a further embodiment of the apparatus shown in Fig. 17A and Fig. 17B, showing a means for inserting and temporarily storing a larger volume balloon then can be easily stored in an insertion tube that will conveniently be inserted into the esophagus of a patient;
Fig. 18A is a side sectional view, in a comparatively reduced scale, of a gastric balloon storage and insertion apparatus useable to dispose a gastric balloon of this disclosure into the stomach of a patient; and
Fig. 18B is a side sectional view of an alternative embodiment of the apparatus shown in Fig. 18A.

### DESCRIPTION OF THE PREFERRED EMBODIMENTS

### (BEST MODES FOR CARRYING OUT THE INVENTION)

The development of the presently disclosed apparatuses and methods included a consideration of the issues of potential ulceration and perforation associated with prior art gastric balloons. As a result, the applicants developed a configuration that has a high probability of avoiding ulceration and perforation. The following disclosure includes reasons why the apparatuses are preferred and an improvement over prior art in that regard. The improvement resulted from applicants studying the design configurations of the prior art devices, then doing an engineering evaluation of how those devices act when inflated in place in the stomach. Such information was correlated to the resultant failure mechanisms that have been reported for the prior art devices, including looking into the physics of the active elements present in the stomach. The apparatus configurations disclosed herein make use of design features or mechanisms (i.e. structures) which potentially accomplish in a novel and simple manner what prior art devices do in a complex way (i.e. varying the inflation volume over time using complex mechanisms).

There is provided according to the present disclosure a gastric insertable balloon apparatus that may be deployed freely in the gastric lumen of an overweight patient. This apparatus does not block food passage through the stomach, for both liquids and chunks of semi-solids, either through the interior of the stomach or along the walls of the stomach cavity. The present apparatus provides for smooth passage of food at the stomach wall and, in a complex alternative embodiment, through a central funnel-like passage through the apparatus.

The apparatus of this disclosure is advantageous in that it does not apply significant force to the walls of the stomach except when the patent has completed a meal, thus minimizing the likelihood of gastric ulceration or perforation. The toroidal shape of the basic embodiment, and sleeved modules of sophisticated embodiments, provide for this beneficial aspect after the balloon apparatus has been inflated and deployed.

The apparatuses of this disclosure also, unlike many apparatus of the prior art, maintain proper positional orientation within the stomach, such that, through normal conditions inside the stomach, including wave spasms and contractions, as well as gravity, the entrance and exit lumens of the stomach are not blocked. The toroidal configuration of the basic embodiment of the apparatus, as well as the more complex alternative embodiments of the apparatus, promote this functional advantage.

In the case of the more complex alternative embodiments of the apparatus herein described, the apparatus maintains its orientation within the stomach such that the tapered funnel shape of the central passage of the balloon are appropriately positioned to transmit radial forces outward toward the stomach wall, to aid in providing a properly timed sensation of "fullness" while still receiving effective treatment.

The present disclosure provides in the balloon a variable satiety reaction mechanism that is tied to the eating and digestion cycle of the patient. This results because, depending on how much food is wedged at any one time inside the central tapered funnel of one preferred embodiment of the apparatus, more or less pressure is created radially outward that causes contact with, and extension of, the stomach wall. This benefit is realized without obstructing the passage of food through the stomach, and without causing ulcerating pressures or perforation corners or edges to injure the stomach lining.

During between-meal periods, the disclosed apparatuses assume the appropriate design size within the stomach, without pressing harshly against the stomach wall. The novel internal structure within the balloon provides for this feature. This type of balloon can be larger than what would lend itself easily to storage within an insertion tube. Although in situ inflation is an option, it is not a necessity, in light of the already inflated nontube-storage balloon insertion apparatus disclosed herein. With the disclosed insertion tool, one can take a large balloon that is inflated outside the patient, and then pass right into the patient's stomach, through a tube that is positioned in the patient's esophagus.

Only after a study of the issues discussed in the above paragraph did it became clear that to avoid the pitfalls of the prior art, and to assure operability without having the failure mechanisms of the prior art, the apparatuses would need to be inflated to a controlled extent such that they provide sufficient structure to resist collapse. Hence, the apparatus would not fold up and seal up the central hole, and would not collect at the entrance or exit to the stomach, and close or plug those openings.

A mode of manufacturing a multi-chambered balloon is disclosed. The internal details of the balloon are important for the proper function of the balloon, in terms of providing the necessary structural rigidity, and as to how inflation and deflation may be accomplished. Those things are not obvious, and were an important design concern.

The inventive apparatus described herein improves upon prior art by improving the geometry of a stomach-insertable apparatus to a more flexible and anatomically accommodating shape. The improved apparatus geometry potentially decreases motility of the apparatus, and reduces the chance of obstruction of the gastric lumen.

A related objective of the present disclosure is to provide a means of inserting and retrieving a gastric apparatus without causing undue inconvenience or risk to the patient and to minimize costs. The invention disclosed herein addresses all of these goals and objectives.

The apparatus of this disclosure is to be distinguished from the various inflatable balloon devices which are interactive tools for use by a surgeon during surgery. Such surgical tools often include as a part of the device a lengthy tubular handle-like element that allows the surgeon to manually deploy the device to the location of interest within a body cavity or lumen. They also permit the surgeon to make positional adjustments in the tool real-time, as the surgeon conducts the procedure. None of these surgical tools would be complete, or functional, without the tubular handle part of the device. The present apparatuses do not feature similar controlling handles.

Also, known balloon-like surgical tools rely for their operation on the surgeon's inflation of the tool in real-time, and maintenance of the amount of inflation, throughout the time the tool is deployed.

Thus this disclosure provides a method and apparatus for placing one or more un-inflated balloons into a tube that will fit the human esophagus, inflating the balloon and thereafter inserting the pre-inflated balloon into the patient. The inflated balloon(s) can be of various internal volumes, and may be inflated with various fluids. A gastric balloon may be stored in the insertion tube until such time as the balloon is to be inserted into a patient. According to the invention, gastric balloons are provided having a designed shape, size, and structure that ensure that, when placed in the stomach, demonstrate advantageous characteristics:
a) A toroidal gastric balloon apparatus resists crushing and folding such that the central openings defined therein will not be closed off by normal stomach muscle flexure, thereby ensuring the maintenance of a food passageway through the balloon, and through the stomach.
b) The shape and placement of the balloon within the stomach ensures that it does not clog the entrance or the exit of the stomach.
c) Between meals when the stomach is void of food, the balloon apparatus does not cause unusual pressure on the stomach wall; this advantage is realized by balloon structural configuration alone, something that prior art patents have attempted to achieve with complex functional systems (i.e. attempt to vary inflation pressure from time-to-time for this purpose); namely, relief to the patient between meals.
d) Some of the balloon apparatuses of this disclosure are of a size that permits the balloon to be inflated after being disposed in an insertion tube, and then stored in that form.
e) It is preferred that balloon size permits the balloon readily to pass through the digestive system when deflated. Balloons that incorporate various types of timed release mechanisms are disclosed, enabling the planned termination of treatment in a benign manner.
f) The gastric balloon in this disclosure is fabricated from thin and very flexible elastic membrane which precludes the features of the prior art which included corners or edges that could potentially cause perforation of the stomach lining.
g) An aspect of the disclosure is a method and apparatus for inserting, using vacuum or fluid pressure forces, an inflated balloon into a storage and insertion tube for storage.

### Intragastric Balloon Apparatus

Historically, balloons used inside the stomach for weight control have been predominately spherical in shape and filled either with air or saline solutions. Variations on this shape have included spherical or slight oval shapes, and an elongated, tubular sleeve, torus-shaped (toroid) balloon. Balloons adapted for this use have always been filled after they were inserted into the stomach, typically to volumes ranging from 200ml to 800ml. Anatomical capacity of an adult stomach is known to range from 1000ml to 2000ml, with some extreme cases going to 5000ml. Because the shape, tonus, and volume of the human stomach is widely variant, any intragastric balloon device must have wide flexibility in application to adequately address the needs of a maximum of patients. A small stomach with a sphere or oval balloon may experience temporary obstruction, while a larger stomach will be able to accommodate a full meal in spite of the presence of a device, especially since most stomachs will easily expand to more than double their volume if necessary. In the former case, there may be retching or vomiting, and in the latter case the efficacy of the device as a weight loss therapy may be limited.

Most prior-art devices, whether inserted through the esophagus or through surgery, are intended to be retrieved from the stomach by first deflating them, then pulling them back out through the same access by which they originally were inserted. There was, therefore, minimal concern about the thickness and elasticity of the balloon material. However, complications ensue when premature or unplanned deflations of known free-floating balloons occur. Complications were particularly consequential to the deflated balloon passing to the small intestine, and there becoming lodged, requiring surgical removal.

It is known that objects as large as 1.5cm or more in diameter readily pass through the adult digestive system without incident. Fluid-filled balloon apparatus disclosed herein exploit this capability of the human digestive system.

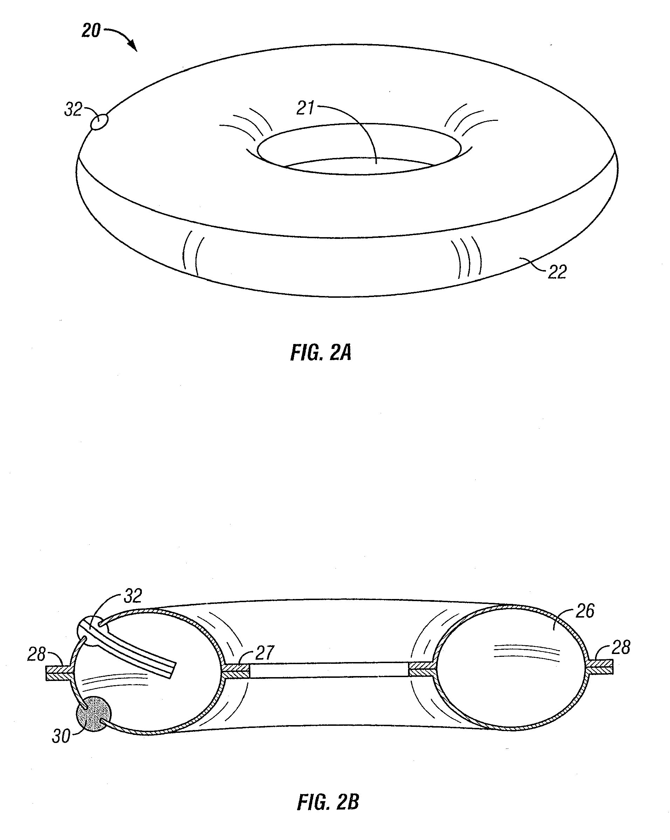

Figs. 2A and 2B illustrate one embodiment of balloon apparatuses according to the present invention. The figures show a toroidal embodiment **20** which is fabricated from biocompatible elastic membrane **22** filled with a sterile fluid **26.** A design component of this embodiment is the capacity to use more than one of these toroidal balloons **20** in the stomach at any one time. However, the use of a single balloon **20** is not precluded. It is anticipated that multiple toroidal balloons **20** will be used in most procedures.

The apparatus **20** includes a thin elastic membrane balloon that has the desirable characteristics of compatibility with use inside the human stomach as well as sufficient elastic stretch capability to expand (within a flexible insertion tube) to the volume it will have after it is inside the stomach. This the apparatus **20** does by stretching and filling an insertion tube volume (not shown in Fig. 2) to sufficient length for required gastric placement volume without failure or leakage. Candidate materials for the membrane **22** include silicone elastic membranes. Candidate materials also include elastic membranes that are biocompatible and bio-dissolvable, such that over time, some portion of the balloon wall will be perforated by stomach acid and will result in pre-planned deflation, dependent on the thickness of the membrane and the selected membrane material. In this disclosure and in the claims, "bio-dissolvable" refers to a solid or semisolid material that is innocuous inside the human stomach, but which dissolves at a know rate in the presence of gastric fluids. Formation and assembly of the balloon may make use of molding, heat sealing, or bonding with adhesives. For example, the apparatus **20** may be manufactured from multiple portions of membrane, having seams **27** sealed by heat, ultrasonic welding, or suitable adhesive.

The fill port **30** is used to inflate the balloon **20** prior to final configuration, and then is sealed through current art methods, such as ultrasonic welding or heat sealing. An alternative port **30** configuration contains a self-sealing fill port which accommodates filling the balloon **20** with a needle (not shown) such as further described later herein. The balloon **20** is filled with a sterile fluid **26,** preferably having an inert coloring constituent that colors the patent's urine or feces to inform the patient in the event of a balloon leak.

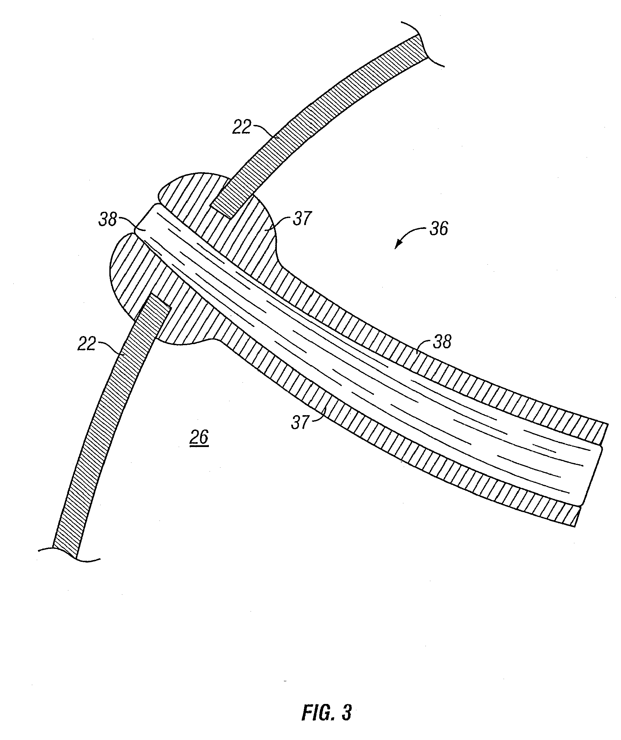

This embodiment of the balloon **20** may feature a time release deflation plug **32,** as further illustrated in Fig. 3. The release plug **32** allows the apparatus to be used as a temporary weight-loss therapy while obviating a second procedure to remove the balloon **20.** After a predetermined time, such as three or six months, the plug **32** loses its ability to retain the fluid **26** contained within the interior of the balloon **20.** At that time, the balloon **20** will deflate and proceed to pass out of the patient through the digestive system. One means of accomplishing the time release is through the action of stomach acid on the time release element **38** of the balloon. As seen in Figs. 2B and 3, a small cartridge **37** is provided through an aperture in the balloon wall, and in sealed conjunction with the membrane **22.** The cartridge **37,** which may be generally tubular, and fabricated from flexible elastic material, contains the time-release plug substance **38.** The plug substance **38** is a biologically innocuous composition which degrades in the presence of stomach acid. The length of time required for the plug **38** to completely dissolve is a function of its precise composition, as well as its diameter and/or length within the cartridge **37.**

The balloon **20** is inserted into the patient's stomach. When the patient begins to eat, the internal stomach volume available for food is reduced by the presence of the balloon **20,** thus precluding the patient from overeating. The inventive balloon designs add the important benefit of enhancing this "fullness effect" by their built-in mechanical advantage reaction mechanisms that cause added radial force and pressure against the internal surfaces of the stomach wall. The apparatus does this while avoiding creation of undue long-term pressure on the stomach wall between meals, to avoid the potential for ulcer-causing mechanisms.

The torus membrane balloon **20** embodiment seen in Figs. 2 and 3 offers several advantages. It is simple, and can be affordably manufactured in a variety of inflated volumes. The apparatus **20** may be readily used in conjunction with the inflation, storage, and insertion apparatus disclosed hereinafter, including use of more than one balloon in one insertion tube.

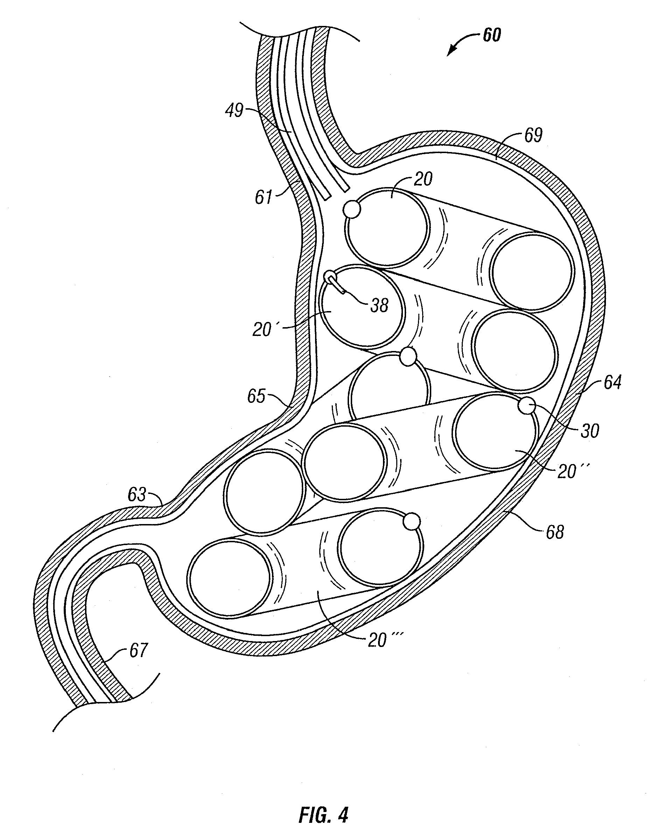

Reference is made to Fig. 4, showing the simultaneous use of (for example only) five individual torus balloons **20, 20', 20", 20'''** in a single patient. The balloons are insertable using the insertion tube **49.** It is noted that the plurality of balloons **20, 20', 20", 20'''** well fills much of the stomach **60** volume between the gullet **61** and the pylorus **63** (the later leading to the duodenum **67.** Under the action of the muscular layer **68** of the stomach, the balloons **20, 20', 20", 20'''** can move about within the stomach **60** (while pressing against the relatively smooth interior mucous membrane **69)** to accommodate the general shape of the stomach **60** between the fundus **64** and the gastric notch 65.

Thus a plurality of torus apparatuses **20** may be used to achieve the desired fill volume for the patient. Additional balloons **20** can be added over time should the patient's stomach volume increase during the course of treatment. In instances where a plurality of apparatus are placed, release times for the balloons can be varied for temporally separated deflation of the balloons present at any one time in the patient's stomach. The torus shape accommodates food passage through the stomach without obstructing the stomach's entrance or exit lumens. The balloon **20** can readily move within the stomach cavity through normal digestive processes, including stomach flexure, to accommodate food passage. Also, the toroidal shape provides structural resistance to deformation. Such a torus-shaped apparatus **20,** when inflated, also presents dimensions large enough to prevent passage of the apparatus through the pylorus as well as reducing the likelihood of obstructing the pyloric antrum, while requiring a minimum of volume when introduced to the stomach. Unlike prior art, this allows sufficiently sized balloons to be filled prior to the procedure, thus reducing the practitioner's requirements during the procedure.

When more than one apparatus **20** is inserted, it is unlikely that the centrally located through-holes **21** (Fig. 2A) will become registered in complete alignment. Thus it is anticipated that the motility will be reduced. Reduced motility increases the time required for solid foods to pass through the stomach to the intestines where it is absorbed, enhancing the efficacy of the apparatus in weight-loss therapy.

The elastic material used to fabricate the balloon membrane **22** can be marked with radiographically opaque material. Such marking aids in monitoring the balloon's presence in the patient and in locating the balloon for retrieval should surgical or endoscopic intervention become necessary.

Upon deflation, either accidental or intentional, the apparatus is anticipated to deflate sufficiently to pass through the patient's intestinal tract without incident. The thinness of the membrane **22** and limited cartridge **37** dimensions in a deflated configuration present a profile significantly smaller than 1.5cm, with the majority of the membrane **22** reduced to a flaccid state. Objects of this size have been shown to easily pass through a healthy bowel.

The present disclosure also offers alternative time-release mechanisms for timed deflation of a gastric balloon. These alternative embodiments offer a release means which does not make use of a patch arrangement on the surface of the membrane **22.**

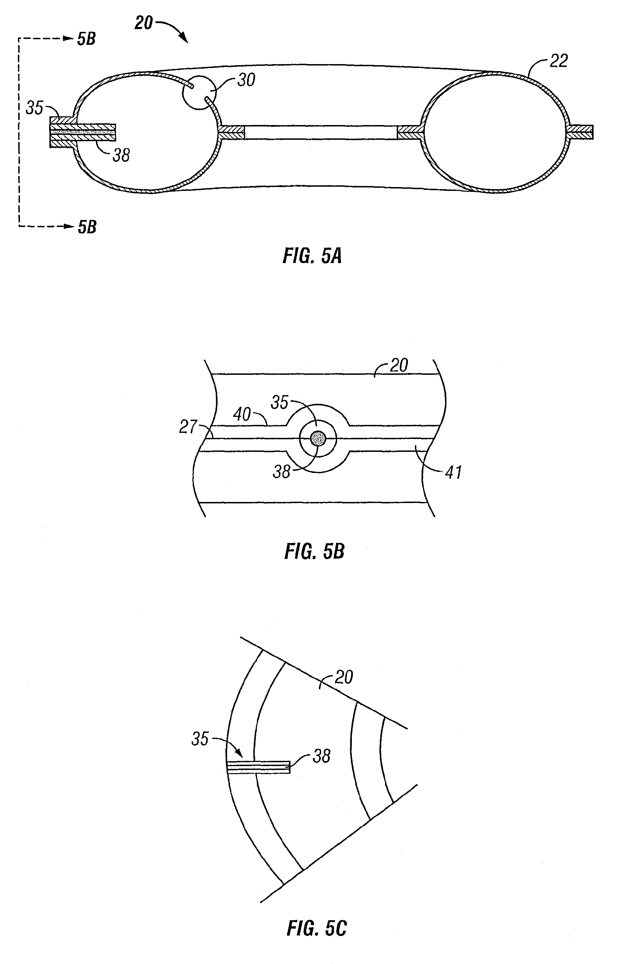

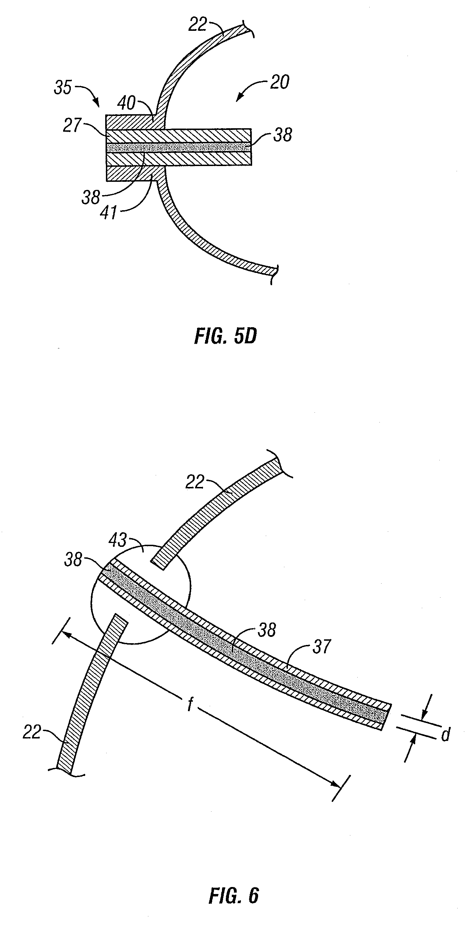

Fig. 5A shows a time release apparatus including a thin flexible elastomeric tube **35** filled with a biodegradable dissolve material **38.** The release time in such an embodiment is controlled by the "fuse length" of the tube **35.** Referring to Figs. 5A-5D, it is seen that the tube **35** is bonded in place within the seam **27** between separate membrane parts **40, 41,** of the balloon **20,** a toroidal balloon being shown for illustration purposes only. One of ordinary skill in the art will appreciate that such a tube-type fuse plug **35** may be employed in the seam **27** of any other style of gastric balloon.

A marked advantage of the present invention is the provision of a time release deflation mechanism that permits a controlled timing of the deflation event, thus the expression of a "fuse." Some known devices in the art include a deflation mechanism, but which offers no selectivity or pre-determination in timing of the deflation event. For example, some devices in the art have a patch-like element affixed to an aperture in the balloon with a bio-absorbable or biodegradable window forming a center of the patch, with the overall depth of the bio-dissolvable material being of similar dimension as the balloon membrane thickness. Such devices make no use of a "fuse length" as a controlling design variable for determining, or setting, the length of time to deflation.

In contrast, the present invention offers a time release deflation mechanism featuring, for example, a long, thin cord of bio-dissolvable material. By predetermining the diameter and/or length of such a plug or fuse, the time of deflation may be predetermined and comparatively closely controlled. Moreover, the bio-dissolvable fuse plugs according to the present disclosure may be small in overall size, and flexible, permitting the balloon of the present invention to be pre-inflated and inserted using a storage and insertion tube, as more fully explained herein.

Fig. 6 illustrates a variation of the time release deflation mechanism of Figs. 3 and 5A. In this alternative embodiment, the features of the tube-type "fuse" are employed. A long tubular cartridge **37** contains a quantity of bio-dissolvable composition **38,** which may fill all (as shown) or only a portion of the length of the tube **37.** The tube **37** is mounted in the membranes **22** of the balloon, as by a durable button **43** adhered in the wall of the balloon **20.** The length f of the "fuse" is indicated. The time release function of the mechanism is selectable by, among other factors, determining the diameter **d** of the bio-dissolvable fuse plug **38,** as well as the actual length f of the bio-dissolvable fuse **38.**

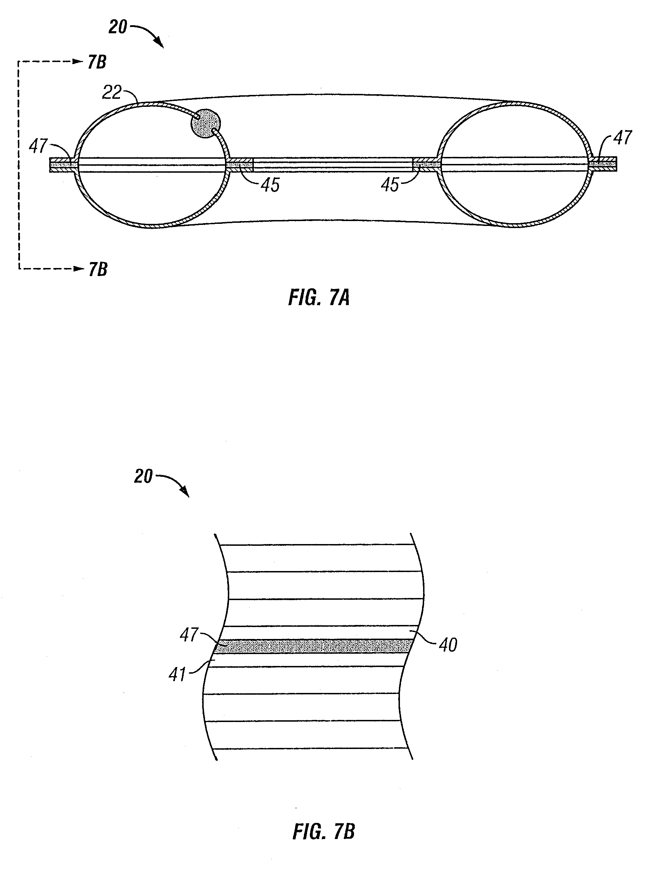

Referring to Figs. 7A-C, an alternative embodiment of the apparatus may use the illustrated time release gasket fuses **45, 47.** This configuration makes use of one or two thin annular gaskets **45** and **47** of biodegradable dissolve material bonded within the inside annular seam **27** or outside annular seam **28,** respectively, between two membrane sections **40, 41** of the toroidal balloon **20.** Thus, the fuses are essentially O-shaped gaskets; the apparatus may incorporate either or both the inner gasket fuse **45** and outer gasket fuse **47.** Again, a "fuse length" f is defined by the radial distance between the inside and outside diameters of either of the gaskets **45, 47** for a given thickness of gasket, and for the type of known dissolve material used.

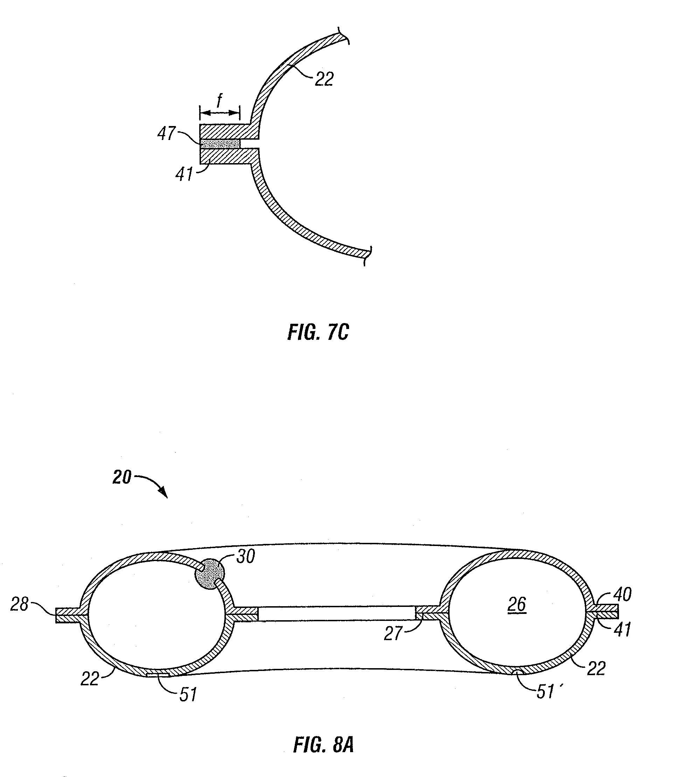

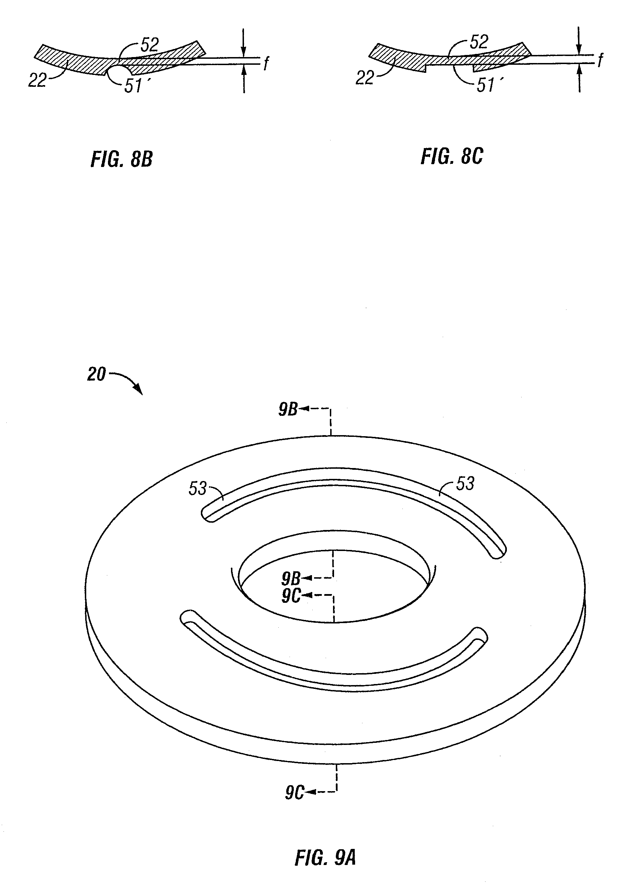

Yet another alternative embodiment of the time release deflation mechanism is depicted in Figs. 8A-C. This embodiment of the timed-release deflator may be called a "dimple fuse." A significant aspect of this embodiment is that the filament or membrane **22** defining the main body of the balloon is, itself, fabricated of a biodegradable elastomer which dissolves in the presence of stomach acid. To this is added a dimple **51,** or dimples **51, 51'** in the surface of the filament **22** which leaves behind a thinned area **52** in the filament, which serves as the pre-planned site (or sites) for the membrane to rupture and deflation to occur. The remaining membrane thickness **52** of the dimple **51** is then the "fuse length" which dissolves first, and results in the release of the sterile fluid contents **26** of the balloon **20.** The dimple profile and the number of dimples **51, 51'** used in a given balloon may vary with the application. Fig. 8B illustrates that a dimple **51** may have a semi-circular profile, while Fig. 8C illustrates a rectangular dimple **51'** profile. A plurality of dimples **51, 51'** increases the probability that the balloon **20** will deflate at the appropriate pre-selected time.

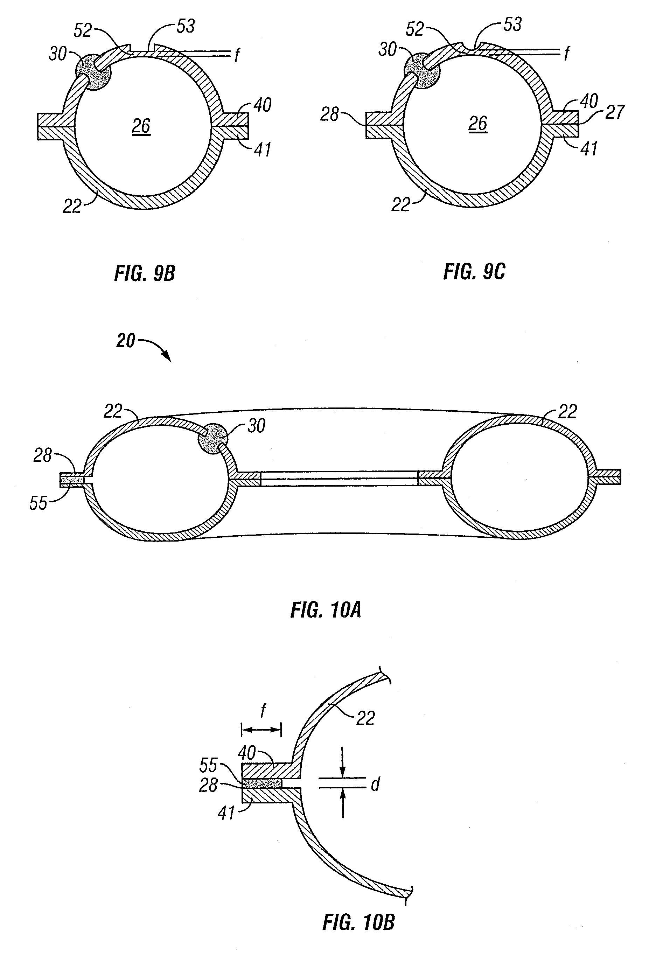

Figs. 9A-C show a time-release groove fuse, which is a variation on the time release mechanism shown in Fig. 8. Rather than dimples, the mechanism of Figs. 9A-C utilizes one or more grooves **53** in the exterior of the membrane **22** defining the body of the balloon **20** (again, a toroidal balloon shown merely by way of example). The "fuse length" is the thickness of the material in the thinned area **52** remaining in the bottom of each groove **53;** thus, the deeper the groove **53,** the shorter the functional life of the balloon within the patient's stomach. The groove profile and length may be varied in different balloons to suit the particular application. Fig. 9B illustrated a groove having a rectangular profile, while the groove **53** of Fig. 9C manifests a curved profile in section. A groove's length increases the probability that the balloon **22** will deflate at the proper time, as a rupture anywhere along the length of the groove **53** will result in timely deflation.

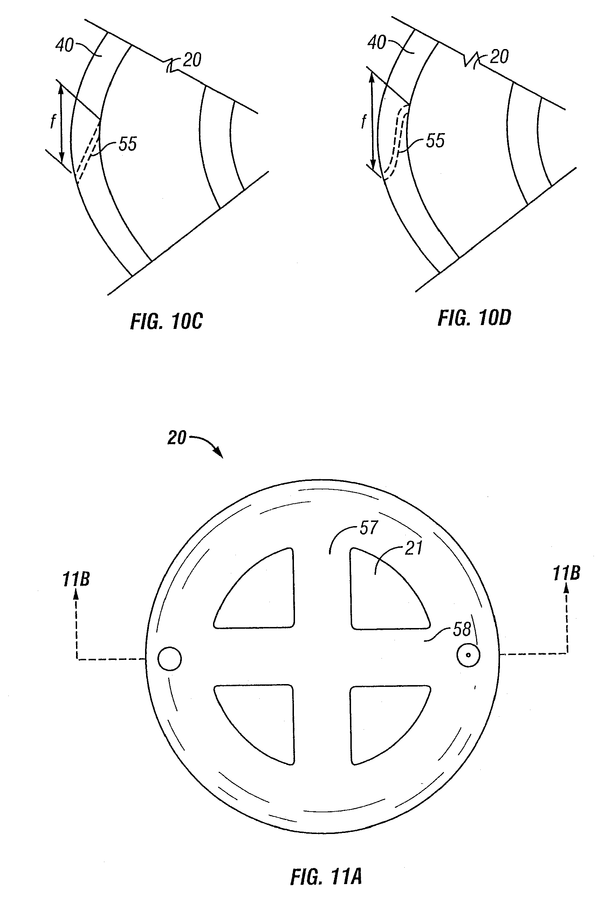

Referring now to Figs. 10A and 10B, there is seen yet another alternative embodiment of the time-release deflation mechanism. A string fuse time-release deflation mechanism is provided. The string fuse **55** makes use of a small-diameter (e.g., cylindrical) prism **39,** made from biodegradable material that is bonded within the seam **28** between sections **40, 41** of the balloon **20,** similar to that for the tube fuse of Fig. 7A. Again, a "fuse length" is defined by the length of the fuse string **39.** Referring to Figs. 10C and 10D, it is seen that the string fuse **55** need not be radially oriented in the seam **28,** and need not be a right cylinder. Rather, the string fuse **55** may have a skewed orientation relative to the torus of the balloon **20,** and/or may have a meandering configuration as seen in Fig. 10D. Alternative embodiments of this version employ a laterally broader or wider rectangular "lozenge" of dissolve material, rather than the relatively narrow string **55** fuse of Figs. 10A-B.

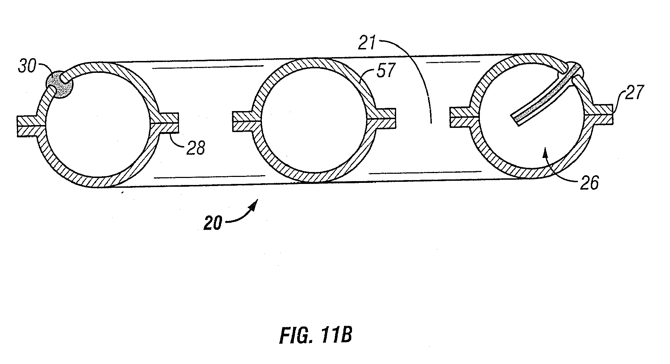

An alternative balloon configuration according to this disclosure, which makes use of inventive structure to ensure shape maintenance inside the stomach, is illustrated in Figs. 11A and 11B. In this embodiment, one or more spokes **57, 58** extend diametrically across the central opening **21** of the torus of the balloon **20** to increase structural stability of the apparatus while still permitting passage of food through the torus.

The disclosure is extended here to balloons which employ structure to ensure that the balloon, once inserted into the stomach, maintains itself within a certain region of the gastric lumen, and fills a greater volume than a single toroidal balloon.

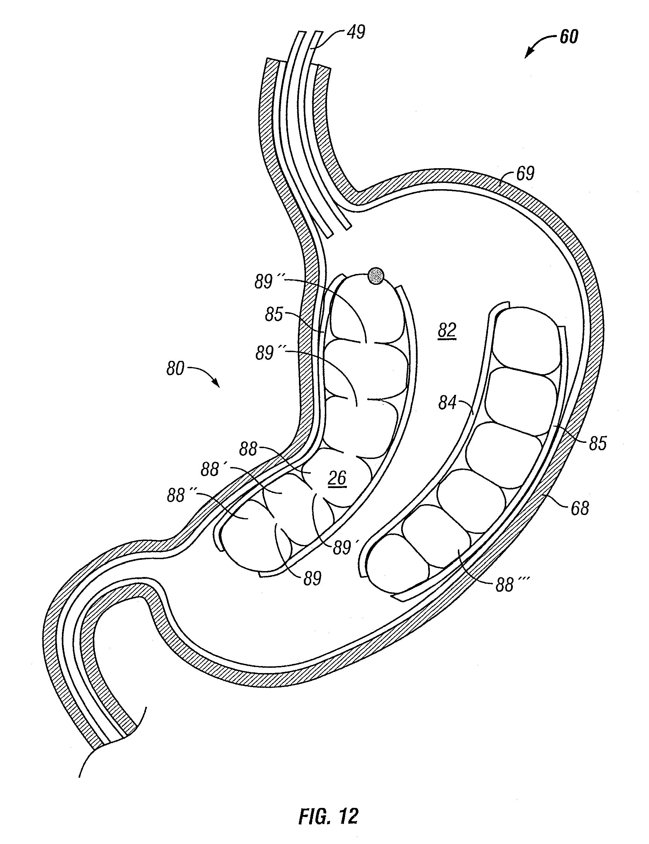

Fig. 12 illustrates another embodiment of a single fluid-filled toroidal funnel balloon **80.** Some of the elements of the basic invention described hereinabove are incorporated in this embodiment, labeled with the same label numerals. In this embodiment, the configuration of the balloon **80** promotes in the patient a feeling of early satiety, thus boosting treatment. It is observed that a series of torus-shaped balloons are connected to define a funnel-shaped apparatus. This may be achieved by providing a series of toroidal balloons having equal exterior diameters but with gradually decreasing internal diameters (proceeding from top of apparatus toward the bottom); alternatively, the exterior diameters may also decrease in correlation with the incrementally decreasing internal diameters of the respective balloons.

Balloon **80** significantly reduces the available volume of the stomach for ingestion of food, while not obstructing the gastric lumen. The desirable characteristic of leakage detection through color marking of the fill fluid **26,** and radiographic identification may be retained. The latter is facilitated by using ultra-thin elastomeric material for balloon fabrication, thereby limiting the volume extent of the balloon when deflated. The balloon **80** may be retrieved through the esophagus in the event it becomes medically indicated.

Further, the general toroidal configuration is maintained to maintain an inner passage **82** for ingested food, and to maintain radial pressure on the stomach's internal mucous lumen **69** to reduce deformation from such actions as the muscular contraction of the stomach wall **68.** The use of a smooth inner sleeve **84** and a smooth outer sleeve **85** on the balloon **80** accommodate smooth passage of food through the stomach; food moves through the inner passage **82** along the funnel-shaped inner sleeve **84.** A series oftorus or donut-shaped modules **88, 88', 88", 88'''** (six shown in Fig. 12) serve to provide structure supporting the inner and outer sleeves **84, 85,** and maintaining them in spaced-apart relation. Thus, the membranes of the respective modules function as internal panels or baffles which lend structural stability and overall definition to the apparatus. Preferably, the interiors of the several modules **88, 88', 88", 88'''** are in fluid communication with one another, via internal ports **89, 89', 89"**. The modules **88, 88', 88"**, **88'''** preferably are manufactured from a flexible elastomeric membrane, such as that previously described herein, and of which the inner and outer sleeves **84, 85** are composed. The broadly curved and rounded outer sleeve **85** has gentle contact with the stomach lining **69,** aiding in prevention of ulceration or other stomach lining difficulties.

The inner and outer sleeves **84, 85** are sealably attached to the uppermost and lowermost modules (i.e., at the entrance and exit of the central passage **82),** thus insuring that food particles do not become lodged in the convolutions or the stacked toroids of the apparatus. Such lodgment of food otherwise may cause harmful stomach bezoars.

Because it may be necessary to fill the larger forms of this type of balloon **80** after it is inserted into the stomach, the apparatus may be intubated (via insertion tube **49)** in a partially or fully deflated state. The medical practitioner may determine that selectively timed deflation would be precluded with this embodiment, to reduce the chance of luminal obstruction due to partial deflation. Extraction through the esophagus could then be used at the appropriate time.

Continued reference is made to Fig. 12. The funnel shape of the central inner sleeve **84** of the balloon **80** offers several advantages. Significantly, the increased contact with the stomach lining **69,** and pressure thereon as food fills the central passage **82,** exploit the natural wedging effect of the funnel-shaped inner sleeve **84.** After food has passed through the central passage **82,** the "wedging effect" due the funnel shape is reduced, and the reduced stomach lining contact and pressure as the food is expelled from the stomach by digestion between meals fostering a sense of well-being in the patient after a meal.

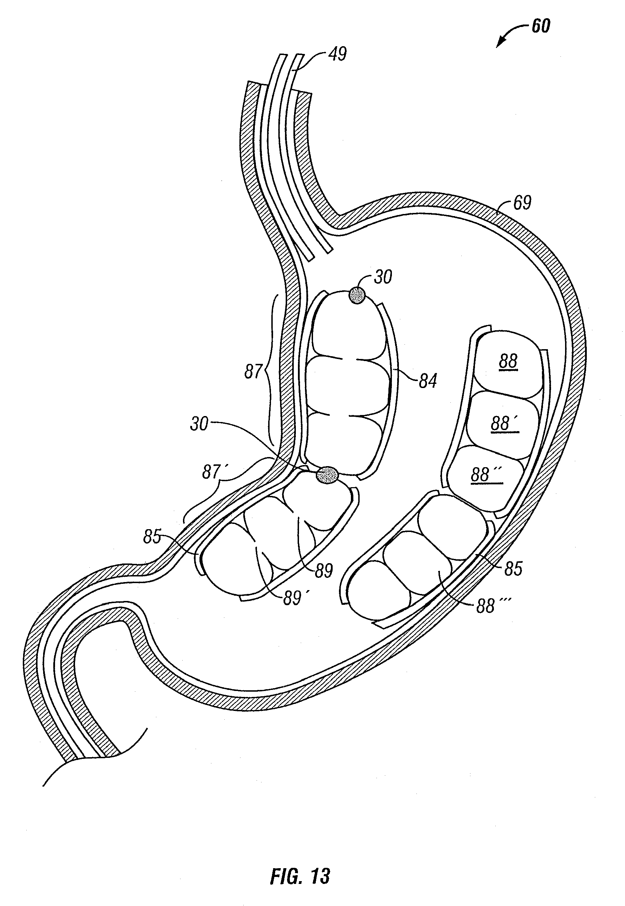

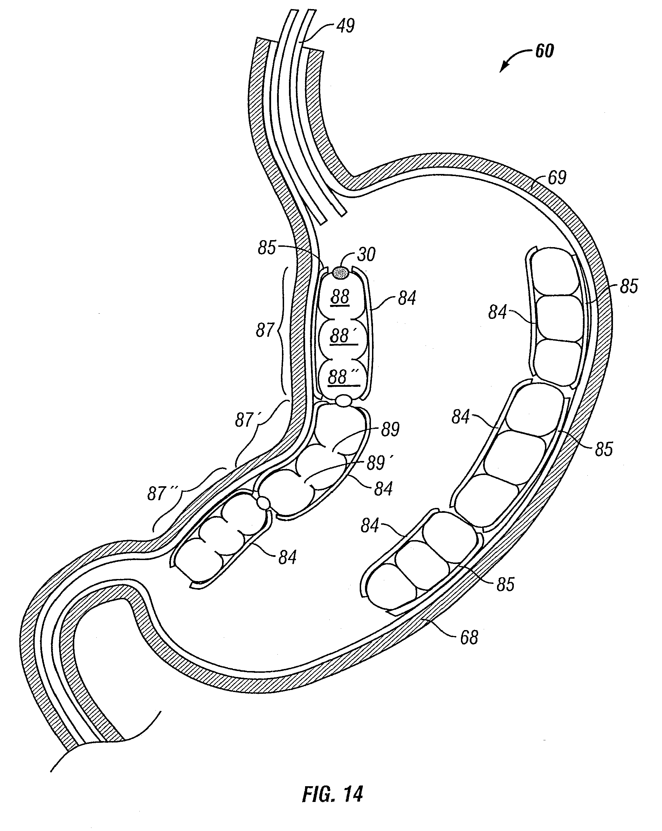

Yet another alternative embodiment of the balloon apparatus **80** is shown in Figs. 13 and 14, a two-staged fluid filled membrane toroidal funnel structure balloon and a three-staged fluid filled toroidal funnel structure balloons, respectively. The drawings illustrate that the type of balloon **80** seen in Fig. 12 may be divided into two sections **87, 87'** (Fig. 13) and three sections **87, 87', 87"** (Fig. 14) are illustrated. Each section includes a plurality (three shown in the figures) of toroidal internal modules **88, 88', 88'''** linked by a common surrounding membrane **22.** The medical practitioner may prescribe one, two, three or more sections as deemed appropriate, the individual segments being separately insertable and removable. Despite being separately manipulated, the individual sections cooperate to define a funnel-like apparatus after the manner of that depicted in Fig. 12.

The use of such segmented apparatuses having shorter sections offers certain advantages. Segmented apparatuses ease tailoring the balloon to specific luminal diameters of the stomach **60,** such as indicated in the drawings. Further, shorter sections accommodate reduced stress on the balloon material during filling within a storage and insertion tube. It should be noted that other inflated structural configuration of the balloons described in this document would remain within the scope of this design disclosure.

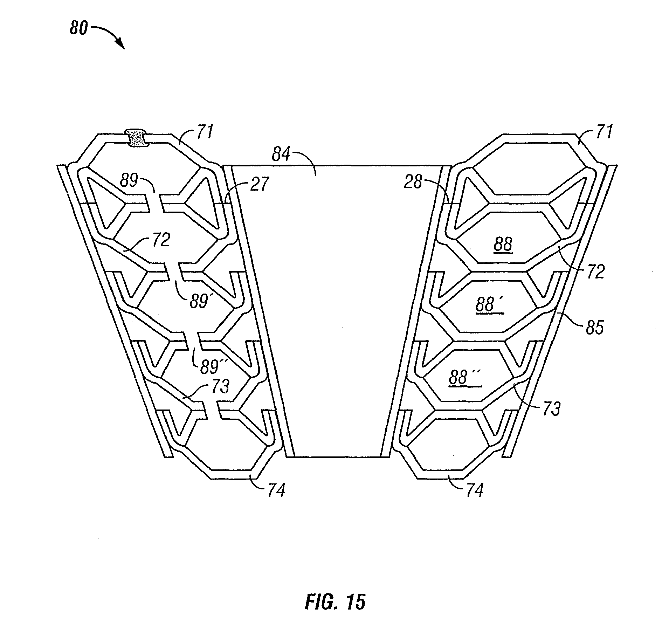

Fig. 15 depicts one possible manufacturing mode and configuration for a funnel-type balloon **80** such as those of Figs. 12 and 14. The balloon **80** may be manufactured from a collection of flexible annular membrane sheets **71, 72, 73, 74,** bent and folded in some cases, as seen in the figure. As configured and joined as seen in Fig. 15, the membrane sheets **71, 72, 73, 74** collectively define and constitute the funnel modules **88, 88', 88'''** as previously described in relation to Fig.12. The individual sheets are joined at seams **27, 27', 28, 28'** as with adhesive, heat sealing, or the like. Selected sheets are perforated with internal port **89, 89'** apertures to provide fluid communication between the interiors of the modules **88, 88', 88'''.**

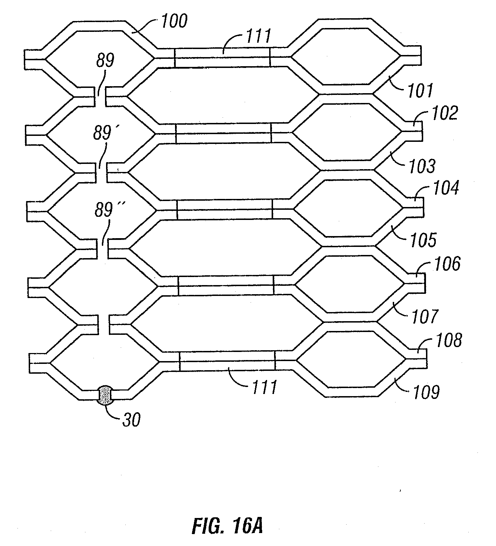

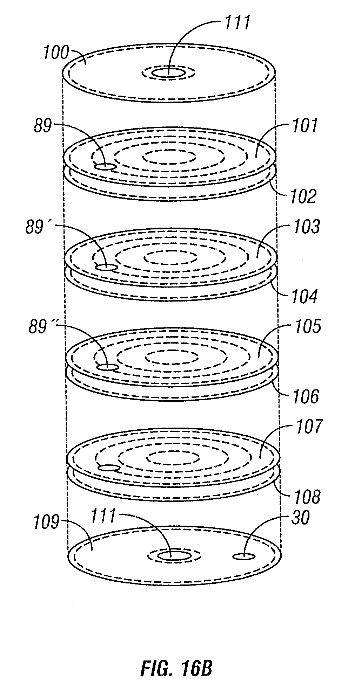

An alternative fabrication mode and configuration of a modular multi-chambered gastric balloon apparatus in accordance with the invention is depicted in Figs. 16A-D. A series of annular membrane sheets **100-109** are cut (for example die-cut from membrane roll stock), stacked, and selectively joined to fashion a somewhat accordion-like gastric balloon apparatus. Fig. 16B illustrates in an exploded (pre-assembly) view that each of the membrane sheets **100-109** is cut to a circular or oval shape. All the sheets **100-109** (ten shown in the figures by way of example) also have a central aperture **111** cut therein. Each of the intermediate sheets, **101-108** is provided with an internal port hole **89** therein radially between the sheet's central aperture **111** and the periphery of the sheet. A first terminal or end sheet **100** does not have an internal port hole. A second terminal or end sheet **109** disposed to define the opposite end of the apparatus is provided with a pluggable fill port **30** through which the sterile fluid is introduced into the balloon interior.

The various sheets **100-109** may be fabricated to have uniform equal diameters, as suggested in the figures, or alternatively may have serially increasing graduated diameters to provide a more horn- or funnel-shaped apparatus. Where the overall diameters of the sheets **100-109** are so serially graduated, the central apertures are correspondingly rationally sized in diameter. It will be readily appreciated that alternative embodiments may feature sheets having uniform external diameters, with only the internal diameters increasing incrementally to define a funnel-shaped central passage.

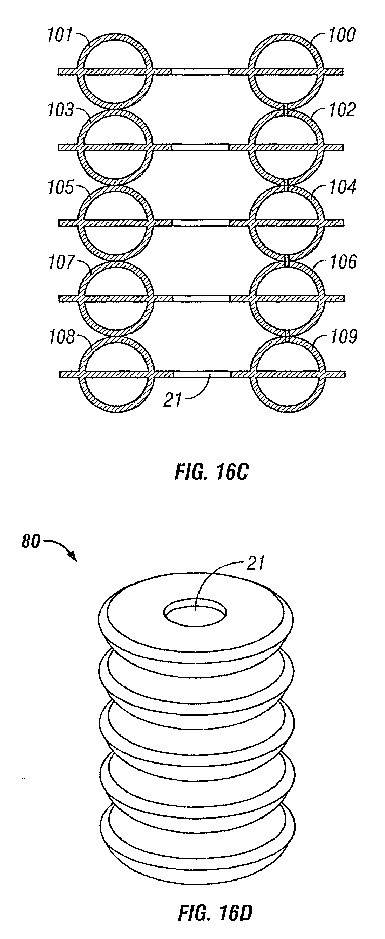

Having particular reference to Fig. 16A, it is seen that the sheets **100-109** may be folded and then stacked in mutual co-registration with their central apertures **111** and internal ports **89** axially aligned. Where adjacent sheets come in contact to define a seam, the generally annular seams **27, 28** are durably conjoined as with suitable adhesive, heat welding, or the like. When the interior of the balloon **80** is inflated with appropriate fluid, the spaces between adjacent sheets increase, and the apparatus expands (mostly axially) to define the functional apparatus **80** seen in perspective in Fig. 16C and in general cross-section in Fig. 16D. The fill port **30** is then closed in accordance with known plug means.

### Balloon Inflation, Storage, and Insertion Device

The inventive toroidal intragastric balloon **20** or **80** may be inserted into the patient's stomach via an insertion tube. This configuration and method obviates all filling, adjustment, and most monitoring tasks currently required in the art. Conventional gastric balloons have various sealing devices that must function correctly, following intragastric filling, for the procedure to be successful. During and after the procedure, the practitioner must observe the device for leaks. Implanted devices have also been known to leak over time. Both of these events reduce the efficacy of known devices. According to the present disclosure, the filling and sealing of a balloon apparatus prior to the procedure permits the apparatus to be inspected for leaks, reducing the likelihood of unintended leakage after deployment.

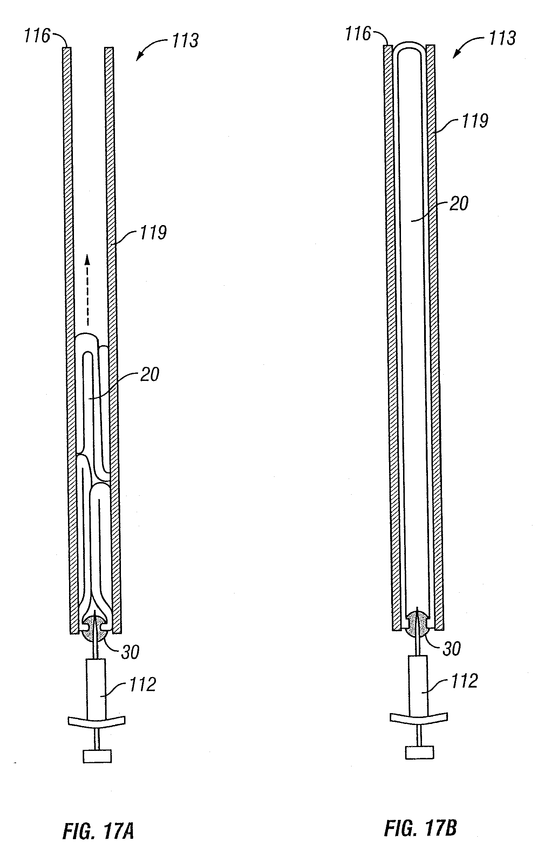

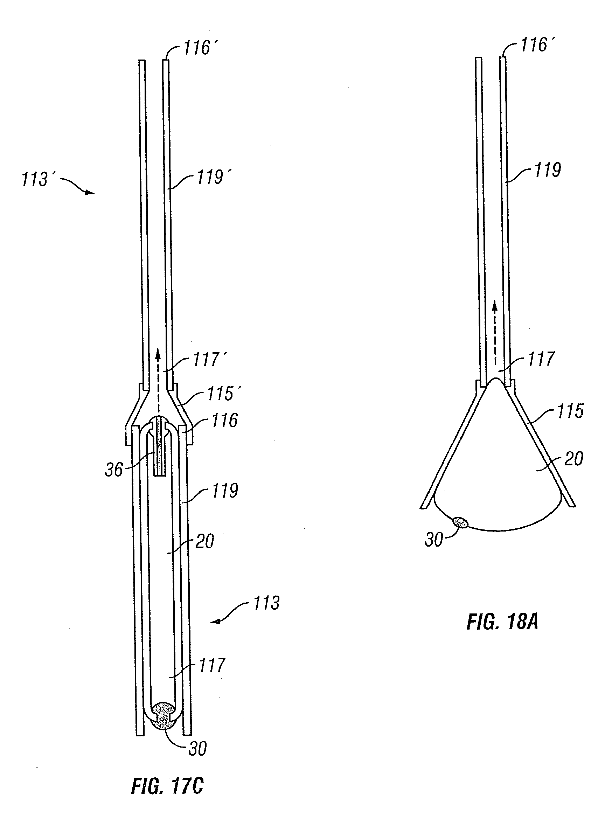

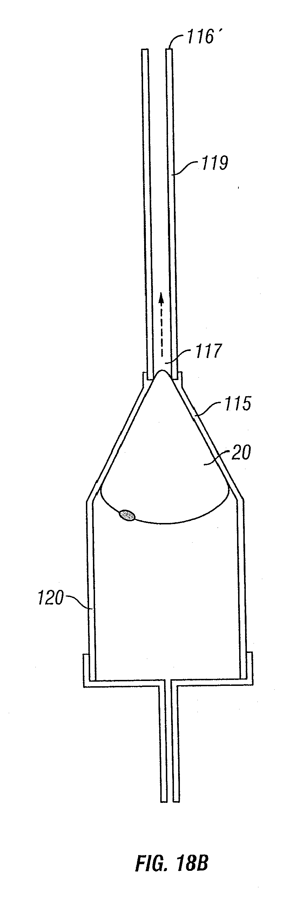

Attention is invited to Figs. 17A, 17B, 18A and 18B. The apparatus and method of the disclosure provide a means and mode for filling an intragastric balloon **20** and storing it prior to use in treatment. Once filled, the balloon apparatus **20** is inserted into a lubricated insertion tube defining the insertion apparatus **113** that also acts as a balloon storage container until the time of use. The thin elastic membrane balloon **20** has the desirable characteristics of being compatible with use inside the stomach, and has sufficient elasticity to expand within a flexible storage and insertion tube apparatus **113** to the treatment volume it will assume after disposition in the stomach. The balloon stretches and fills the insertion tube volume to sufficient length for the required placement volume without failure or leakage. Candidate materials include silicone elastomers. The apparatus may then be used to facilitate the deployment of the pre-inflated balloon **20** into the patient's stomach.

Figs. 17A and 17B show that the storage and insertion apparatus **113** features a tube **119** of biocompatible material sufficiently flexible to navigate the anticipated curvatures of the upper gastrointestinal tract (e.g., esophagus) of a sedated patient. The tube **119** preferably features graduated markings on its exterior surface to assist the practitioner in properly delivering the device to the stomach. Removable end caps and protective packaging (not shown) may be utilized to promote stability of the tube **119** and the contained balloon **20** during shipment and storage.

Still referring to Figs 17A-B, the balloon storage and insertion apparatus **113** includes a flexible tube **119** passable through the esophagus, and having length sufficient to obtain the stomach. The length and associated internal volume of the insertion apparatus **113** are sized to accommodate the volume of the balloon **20** to be inserted into the patient's stomach.

The apparatus **113** also includes a needle syringe means **112** for filling the balloon **20** as illustrated in Fig. 17A. The selected uninflated balloon **20** is disposed into the tube **119.** The sterile fill fluid then is injected by the syringe **112** into the balloon **20** via the closeable fill port **30.** The balloon is then filled with fluid to the proper inflated condition seen in Fig. 17B, at which time it is ready for use. Filling of the elastic membrane balloon **20** can be facilitated by the use of lubricants and vacuum at the distal end **116** of the tube **119.** The inflated balloon **20** may then be stored in the apparatus **113** until the time of use. In use, the insertion apparatus **113** is delivered down the esophagus until its distal end **116** is situated at the medically appropriate position in the upper stomach. The pre-inflated balloon **20** (Fig. 17B) is then pushed from the tube **119** and into the patient's stomach. An optional part of the apparatus **113** (not shown in the drawing) is a pusher plunger which moves inside the flex tube **119** to force the balloon **20** to a specific placement in the stomach. The medical practitioner's judgment in this regard may be informed by visible graduations on the plunger, and/or ultrasound imaging, if desired. Alternatively fluid pressure could be used to assist in the movement of the balloon within the insertion tube during the insertion procedure.

The storage and insertion apparatus **113** permits simple insertion of the balloon with minimal discomfort to the patient. Insertion may be performed on an outpatient basis by a medical provider. A large number of preassembled storage and insertion apparatuses, containing filled balloons, may be stored and transported in bulk quantities, and in a variety of balloon shapes and volumes. An advantage thus is the easy sizing of preassembled insertion apparatuses **113** containing filled balloons of various volumes, for selection by the medical provider to accommodate the needs of a given patient. The invention also offers substantial elimination of health risk complications for the patient and reduction of the skill levels required of the medical provider, compared to that associated with previous balloon inflation and insertion systems.

As an example of a dimensional configuration to accommodate an insertion volume, it is observed that an insertion apparatus **113** with a 15mm internal diameter could house a balloon **20** of 83ml capacity in a length of about 0.5 meter. Hence, three such tubes could provide 250ml fill volume in balloons.

Fig. 17C shows a further aspect of the disclosure wherein a means for inserting and temporarily storing a larger size balloon **20** then will conveniently fit into a reasonably short insertion tube **119** with a size that would fit into the patient's esophagus. Fig. 17C shows a large volume balloon 20 in place in a large diameter temporarily storage tube **119,** which is fitted to a connecting piece **115',** that joins the large diameter storage tube **119** to the smaller insertion tube **119'** prior to final placement of the balloon in the patient's stomach. Insertion tube **119'** is sized to fit the patient's esophagus. A plunger (not shown) and/or fluid pressure is used by the practitioner to deliver the balloon **20** into the stomach at the time of the insertion. Connecting piece **115'** provides a sealed connection between the storage tube **119** and the insertion tube **119'.** Those skilled in the art will note that additional larger sized temporary storage tubes can be cascaded, use of additional connecting pieces, to achieve a progressive reduction to a size appropriate for insertion in the patient's esophagus.

Procedures and tools are shown in Figs. 18A and 18B for disposing the inflated balloon **20** into the balloon inflation, storage, and insertion apparatus **113** (Figs. 17A-B). Two forms of the apparatus useable for getting the balloon **20** into the storage and insertion apparatus **113** are illustrated. Fig. 18A shows the placement of a pre-inflated balloon **20** into a funnel tool **115.** Funnel tool **115** is removably engageable to the proximate end **117** of the storage and insertion apparatus **113.** The temporary attachment of the funnel tool **115** to the storage and insertion apparatus **113** provides a sealed connection between the two. The pre-inflated balloon **20** is situated in the funnel tool **115.** Vacuum is then applied to the distal end **116** of the storage and insertion apparatus **113,** which then draws by suction the balloon **20** into the lubricated tube of the apparatus **113.**

The apparatus and method illustrated in Fig. 18B is similar to those of Fig. 18A except that a pressure chamber **120** is associated with the funnel tool **115.** To dispose the balloon into the storage and insertion apparatus **113,** air or fluid pressure in the chamber **120** is elevated (e.g. by pump (not shown)) to push the balloon **20** into the apparatus **113.** The supplied fluid pressure in chamber **120** may replace, or preferably complement, the vacuum applied to the distal end **116** to draw the balloon **20** into the tube of the storage and insertion apparatus **113.**

The intragastric balloon **39** is deployed by the practitioner by a pushing action from the proximal end. This may be through a mechanism located at the proximal end of the insertion tube such as a mechanical push rod or other means that could be assembled to the insertion tube as an integral part of the fully assembled configuration or may be a separate device. The internal pressure of the contained balloon and lubricated surface of the tube interior allow for the deployment of the balloon with a minimum of force.

An additional use for the disclosure of Fig. 18B is as a means of inserting a balloon that has been inflated outside the stomach directly into the stomach without the intermediate step of storing the inflated balloon inside an inflation, storage, and insertion apparatus **113.** Direct insertion of an inflated balloon using the apparatus includes starting the balloon into the flexible insertion tube **119** up to the distal end of the tube **119,** using proximal pressure on the back side of the balloon and vacuum at the distal end **116** of the tube. The vacuum is then removed from the distal end **116** and the distal end of the tube **119** inserted through the esophagus to the depth required for balloon placement inside the stomach. Proximal pressure would then push the inflated balloon through the apparatus **113** and into the stomach. Once the proximal end of the balloon is inside the tube **119** of the insertion apparatus **113,** a mechanical pusher plunger (not shown) may be used for final placement of the balloon inside the stomach.

Although the invention has been described in detail with particular reference to these preferred embodiments, other embodiments can achieve the same results. Variations and modifications of the present invention will be obvious to those skilled in the art and it is intended to cover all such modifications and equivalents.

## Claims

1. An apparatus for insertion into the stomach for the treatment of overweight comprising:
an inflatable toroidal balloon comprising at least one flexible bio-dissolvable membrane, whereby said membrane dissolves after a pre-selected period of time in the stomach, thereby causing deflation of said balloon; and
a sealable port in said membrane for injecting fluid into said balloon.

2. An apparatus according to claim 1 wherein said balloon comprises two annular membrane sections joined at their respective inside and outside circumferences to define inner and an outside seam.

3. An apparatus for insertion into the stomach for the treatment of overweight comprising:
an inflatable toroidal balloon comprising at least one flexible membrane;
a sealable port in said membrane for injecting fluid into said balloon; and
a time-release deflation mechanism comprising a bio-dissolvable fuse plug, whereby said fuse plug dissolves after a pre-selected period of time in the stomach, thereby causing deflation of said balloon.

4. An apparatus according to claim 3 wherein said balloon comprises two annular membrane sections joined at their respective inside and outside circumferences to define an inner seam and an outer seam.

5. An apparatus according to claim 3 wherein said time-release deflation mechanism comprises:
a cartridge disposed through and in sealed conjunction with said membrane; and
a bio-dissolvable plug substance disposed in said cartridge.

6. An apparatus according to claim 3 wherein said time-release deflation mechanism comprises:
a flexible tube disposed through said membrane and sealed thereto;
a bio-dissolvable plug substance disposed within said tube.

7. An apparatus according to claim 4 wherein said time-release deflation mechanism comprises:
a tube bonded in place within one of said seams between separate membrane sections; and
a bio-dissolvable plug substance disposed within said tube.

8. An apparatus according to claim 4 wherein said time-release deflation mechanism comprises a bio-dissolvable substance disposed in one of said seams.

9. An apparatus according to claim 4 wherein said time-release deflation mechanism comprises two annular gaskets comprising a bio-dissolvable substance, one of said gaskets disposed in each of said seams.

10. An apparatus according to claim 3 wherein said membrane comprises a bio-dissolvable substance, and said time-release deflation mechanism comprises at least one dimple in the exterior surface of said balloon.

11. An apparatus according to claim 3 wherein said membrane comprises a bio-dissolvable substance, and said time-release deflation mechanism comprises at least one groove in the exterior surface of said balloon.

12. Apparatus according to claim 4 wherein said time-release mechanism comprises a string of bio-dissolvable substance disposed in one of said seams and extending between the interior and exterior of said balloon.

13. An apparatus according to claim 1 further comprising at least one inflatable spoke across the central opening in said toroidal balloon.

14. An apparatus according to claim 3 further comprising at least one inflatable spoke across the central opening in said toroidal balloon.

15. An apparatus according to claim 1 comprising a plurality of said toroidal balloons serially arranged in contiguous contact and with the balloons' central openings in axial alignment to define an inner passage.

16. An apparatus according to claim 15 further comprising:
an inner sleeve, disposed in and running substantially the length of said inner passage and in contact with said balloons; and
an outer sleeve disposed around and in contact with said plurality of balloons.

17. An apparatus according to claim 15 wherein said plurality of toroidal balloons comprise a plurality of toroidal balloons of increasing inside and outside diameters, thereby defining a generally funnel-shaped apparatus.

18. An apparatus according to claim 15 further comprising a plurality of internal ports between adjacent membranes of said plurality of toroidal balloons thereby providing fluid communication between adjacent balloons.

19. An apparatus according to claim 15 wherein said plurality of toroidal balloons comprises:
a plurality of annular membrane sheets cut, stacked with central openings axially aligned to define a central passage, and selectively joined near the respective inner and outer peripheral edges of adjacent membrane sheets.

20. An apparatus according to claim 3 comprising a plurality of said toroidal balloons serially arranged in contiguous contact and with the balloons' central openings in axial alignment to define an inner passage.

21. An apparatus according to claim 20 further comprising:
an inner sleeve, disposed in and running substantially the length of said inner passage and in contact with said balloons; and
an outer sleeve disposed around and in contact with said plurality of balloons.

22. An apparatus according to claim 20 wherein said plurality of toroidal balloons comprise a plurality of toroidal balloons of incrementally increasing inside and outside diameters, thereby defining a generally funnel-shaped apparatus.

23. An apparatus according to claim 20 further comprising a plurality of internal ports between adjacent membranes of said plurality of toroidal balloons thereby providing fluid communication between adjacent balloons.

24. An apparatus according to claim 20 wherein said toroidal balloons comprises:
a plurality of annular membrane sheets cut, stacked with central openings axially aligned to define a central passage, and selectively joined near the respective inner and outer peripheral edges of adjacent membrane sheets.
